# EUROPÄISCHE PATENTSCHRIFT

(11) **EP 1 696 869 B1**
(45) Veröffentlichungstag und Bekanntmachung des Hinweises auf die Patenterteilung: **19.08.2009**
(21) Anmeldenummer: 04765153.4
(22) Anmeldetag: 08.09.2004
(51) Int. Cl.: A61K 8/35, A61K 8/97, A61Q 19/04, A61Q 17/04

(54) **KOSMETISCHES BRÄUNUNGSMITTEL AUF BASIS VON DIHYDROXYACETON**
COSMETIC BRONZING AGENT BASED ON DIHYDROXYACETONE
PRODUIT BRONZANT COSMETIQUE A BASE DE DIHYDROXYACETONE

(30) Priorität: 09.09.2003 DE 10342369
(43) Veröffentlichungstag der Anmeldung: 06.09.2006
(73) Patentinhaber: Coty B.V., 2031 CC Haarlem (NL)
(72) Erfinder: GOLZ-BERNER, Karin, MC-98000 Monaco (MC); ZASTROW, Leonhard, MC-98000 Monaco (MC)
(74) Vertreter: Walter, Wolf-Jürgen
(86) Internationale Anmeldenummer: PCT/EP2004/010235
(87) Internationale Veröffentlichungsnummer: WO 2005/025531

(56) Entgegenhaltungen:
- WO-A-97/25970
- DE-A- 19 857 010
- US-A- 5 229 104

## Beschreibung

Die Erfindung betrifft ein neues kosmetisches Bräunungsmittel auf der Basis von Dihydroxyaceton.

Die Verwendung von Dihydroxyaceton (DHA) als Bräunungsmittel für die menschliche Haut ist seit Jahren bekannt. Dabei ist DHA mit verschiedenen Begleitstoffen formuliert worden, um die normalerweise diesem Produkt innewohnenden Nachteile auszugleichen. Es ist bekannt, Selbstbräunungsmittel auf Basis von Dihydroxyaceton (DHA) in Form von verschiedenen flüssigen Emulsionen oder Gelen herzustellen. In vielen Fällen bedarf es jedoch der Stabilisierung durch andere Zusatzstoffe. In der WO96/31190 werden Kombinationen von DHA mit Lecithinen und gegebenenfalls Sterolen zwecks Stabilisierung in wässriger Lösung erwärmt und anschließend liposomal verkapselt. Weiterhin beschreibt die WO 01/70186 einen kosmetischen Puder, der DHA, einen Mahakanni-Extrakt und einen Tulpenextrakt enthält.

Aus der EP 954277 ist ein kosmetisches Selbstbräunungsmittel bekannt, das neben bestimmten UV-Filtern in Liposomen verkapseltes DHA und freies DHA enthält und eine natürliche Hautbräunung erreichen soll.

Der Erfindung liegt die Aufgabe zugrunde, ein kosmetisches Bräunungsmittel bereitzustellen, dessen Bräunungswirkung sehr gleichmäßig, intensiv und langanhaltend ist.

Das neue kosmetisches Bräunungsmittel enthält 0,1 bis 4,5 Gew-% in eine lamellare Struktur eingelagertes Dihydroxyaceton (DHA) und 0,15 bis 3,5 Gew-% freies Dihydroxyaceton, wobei das Verhältnis zwischen lamellar eingelagertem DHA und freiem DHA im Bereich von 1:1 5 bis 1:0,25 liegt, 0,01 bis 10 Gew-% Phospholipide 0,01 bis 5 % Gew.-% eines alkoholischen Extraktes von Orangenschalen im Gemisch mit 0,5 bis 8 Gew-% eines alkoholischen Extraktes von Tulpen und bis 100 Gew-% kosmetische Hilfsstoffe, Trägerstoffe, weitere Wirkstoffe und Gemische davon, wobei alle Anteile auf das Gesamtgewicht des Bräunungsmittels bezogen sind.
Vorzugsweise enthält es 0,1 bis 1,5 Gew-% in eine lamellare Struktur eingelagertes DHA und 0,15 bis 2,5 Gew-% freies DHA.

Das lamellar eingelagerte DHA kann ein Produkt sein, das mit Phospholipiden wie Phosphatidylcholin, die eine solche lamellare Struktur ausbilden können, zusammen verarbeitet wird. Dabei können zusätzlich Hilfsstoffe mit herangezogen werden, wie Wasser, Alkohole, z.B. Glycerin, Pentylenglycol, sowie Öle wie z.B. Neutralöl, Shea Butter, Squalan oder auch Ceramid III.

Die lamellare Struktur ergibt sich durch ein spezielles Hochdruck-Homogenisierungsverfahren, wobei DHA in die Lamellen in sehr feiner Verteilung eingelagert wird. Diese Herstellung mittels Phospholipiden, Alkohol, Lipiden und Wasser unter Erwärmung ist z.B. aus WO02/089770 Beispiel 1-3 bekannt.

Durch die Kombination der lamellar eingelagerten DHA-Teilchen mit den freien DHA-Teilchen tritt nicht nur eine gewisse zu erwartende Bräunungsvertiefung auf sondern auch ein Langzeiteffekt. Unter Langzeiteffekt ist eine anhaltende Bräunung für wenigstens 5 Tage, vorzugsweise wenigstens 7 Tage nach einmaligem Auftragen zu verstehen.

Im Gegensatz zu Liposomen-verkapseltem DHA in EP 954277 wird anstelle der Verbesserung des natürlichen Aussehens der Haut nach Hauttönung die langanhaltende Wirkung verstärkt.

Als Phospholipide können z.B. eingesetzt werden Phosphatidylcholin, Phosphatidylethanolamin, Phosphatidylinositol, Phosphatidylserin, Phosphatidsäure, Lysolecithine und Sphingolipide sowie Gemische davon. Bekannte Produkte sind beispielsweise Phospholipon®.

Das neue Bräunungsmittel enthält ein alkoholisches Extrakt von Orangenschalen im Bereich von 0,01 bis 5 Gew-%. Ein solcher Extrakt wird bevorzugt mit Propylenglycol als Extraktionsmittel bei einer Temperatur bis 40 °C gewonnen.

Das neue Bräunungsmittel enthält gleichzeitig ein alkoholisches Extrakt von Tulpen im Bereich von 0,5 bis 10 Gew-% enthalten. Ein solcher Extrakt wird ebenfalls bevorzugt mit Propylenglycol als Extraktionsmittel bei einer Temperatur bis 60°C gewonnen.

Der Zusatz der Extrakte erfolgt vorteilhaft in getrockneter Form, so dass die o.g. Anteile von Orange und Tulpe ausgehend vom Trockengewicht des Extraktes auf das Gesamtgewicht des Mittels bezogen sind.

In einer weiteren Ausführungsform der Erfindung kann dem erfindungsgemäßen Mittel ein Süßholzextrakt (Liquorice) mit einem Anteil von 0,001 bis 1 Gew-% zugesetzt werden.

Das erfindungsgemäße Mittel enthält weiterhin kosmetische Hilfs- und Trägerstoffe, wie sie üblicherweise in solchen Zubereitungen verwendet werden, z.B. Wasser, Konservierungsmittel, Farbstoffe, Pigmente mit färbender Wirkung, Verdikungsmittel, Duftstoffe, Alkohole, Polyole, Ester, Elektrolyte, Gelbildner, polare und unpolare Öle, Polymere, Copolymere, Emulgatoren, Wachse, Stabilisatoren, wobei das Vorhandensein von Emulgatoren, Konservierungsmitteln, Paraffin und Farben möglichst vermieden wird, d.h. Formulierungen ohne die zuletztgenannten Stoffe sind bevorzugt.

Das erfindungsgemäße Mittel kann als Gel oder Emulsion oder Gel-Emulsion zur Anwendung kommen. Dafür können übliche Gelbildner oder Emulgatoren eingesetzt werden.

Zusätzliche Wirkstoffe können sein z. B. anorganische und organische Lichtschutzmittel, Radikalfänger, Feuchthaltemittel, Vitamine, Enzyme, pflanzliche Wirkstoffe, Polymere, Melanin, Antioxidationsmittel, entzündungswidrige natürliche Wirkstoffe, mit Sauerstoff beladene asymmetrische lamellare Aggregate gemäß WO 94/00109; Aufschlussprodukte von Hefen oder pflanzlichen Stoffen, hergestellt durch ein schonendes Ultraschall-Aufschlussverfahren gemäß WO 94/13783, Kaolin sowie mit SiO₂ modifiziertes Kaolin gemäß WO94/17588.

Eine weitere bevorzugte Ausführungsform der Erfindung besteht darin, dem Bräunungsmittel eine Wirkstoffzubereitung mit hohem Radikalschutzfaktor zuzusetzen, die aus folgenden Bestandteilen besteht: einem durch Extraktion der Rinde von Quebracho blanco und nachfolgender enzymatischer Hydrolyse gewonnenem Produkt, das wenigstens 90 Gew- % Proanthocyanidin-Oligomere und höchstens 10 Gew-% Gallussäure enthält, in Mikrokapseln, sowie einem durch Extraktion gewonnenen Seidenraupenextrakt, der das Peptid Cecropine, Aminosäuren und ein Vitamingemisch enthält, und einem nichtionischen, kationischen oder anionischen Hydro-Gel oder Gemisch von Hydro-Gelen, und einem oder mehreren Phospholipiden, und Wasser gemäß WO99/66881 z.B. Wirkstoffkomplex gemäß Beispiel 1 oder 2 oder WO 01/26617, z.B. Wirkstoffkomplex gemäß Beispiel mit weiteren Zusätzen.

Es können weiterhin zusätzlich Erweichungsmittel oder Feuchthaltemittel oder Gemische davon enthalten sein.

Als Erweichungsmittel können normalerweise eine Vielzahl von Verbindungen eingesetzt werden, wie Stearylalkohol, Glycerylmonoricinoleat, Glycerylmonostearat, Propan-1,2-diol, Butan-1,3-diol, Cetylalkohol, Isopropylisostearat, Stearinsäure, Isobutylpalmitat, Isopropylmyristat, Isopropylpalmitat, Oleylalkohol, Isopropyllaurat, Decyloleat, Octadecan-2-ol, Isocetylalkohol, Cetylpalmitat, Siliconöle wie Dimethylpolysiloxan, Polyethylenglycol, Lanolin, Kakaobutter, pflanzliche Öle wie Maisöl, Baumwollsamenöl, Olivenöl, mineralische Öle, Butylmyristat, Palmitinsäure usw.

Als Feuchthaltemittel sind bevorzugt Glycerin, Butylenglycol, Propylenglycol und Gemische davon.

Zu Antioxidationsmitteln gehören Vitamine wie Vitamin C und Derivate davon, beispielsweise Ascorbylacetat, -phosphat und -palmitat; Vitamin A und Derivate davon; Folsäure und deren Derivate, Vitamin E und deren Derivate, wie Tocopherylacetat; Flavone oder Flavonoide; Aminosäuren, wie Histidin, Glycin, Tyrosin, Tryptophan und Derivate davon; Carotinoide und Carotine, wie z.B α-Carotin, β-Carotin; Harnsäure und Derivate davon; α-Hydroxysäuren wie Citronensäure, Milchsäure, Apfelsäure; Stilbene und deren Derivate; sowie Granatapfelextrakte.

Es ist weiterhin vorteilhaft, den erfindungsgemäßen Zusammensetzungen entsprechende wasser- und/oder öllösliche UVA- oder UVB-Filter oder beide zuzusetzen. Zu vorteilhaften öllöslichen UVB-Filtern gehören 4-Aminobenzoesäure-Derivate wie der 4-(Dimethylamino)-benzoesäure-(2-ethylhexyl)ester; Ester der Zimtsäure wie der 4-Methoxyzimtsäure(2-ethylhexyl)ester, Benzophenon-Derivate wie 2-Hydroxy-4-methoxybenzophenon; 3-Benzylidencampher-Derivate wie 3-Benzylidencampher.

Bevorzugte öllösliche UV-Filter sind Benzophenone-3, Butyl-Methoxybenzoylmethane, Octyl Methoxycinnamate, Octyl Salicylate, 4-Methylbenzylidene Camphor, Homosalate und Octyl Dimethyl PABA.

Wasserlösliche UVB-Filter sind z.B. Sulfonsäurederivate von Benzophenon oder von 3-Benzylidencampher oder Salze wie das Na- oder K-Salz der 2-Phenylbenzimidazol-5-sulfonsäure.

Zu UVA-Filtern gehören Dibenzoylmethan-Derivate wie 1-Phenyl-4-(4'-isopropylphenyl)propan-1,3-dion.

Die Verwendung der erfindungsgemäßen kosmetischen Zusammensetzungen kann z.B. erfolgen in Form von Sonnencremes, Sonnengelen, After-sun-Produkten, Pre-sun-Produkten, Tagescremes, Nachtcremes, Masken, Körperlotionen, Körperpuder, Augenkosmetik und in Produkten der dekorativen Kosmetik wie Lippenstiften, Gelen, Lidschattens, Kompaktpuder oder Kompaktwachs, Rouge, Grundierung, Make-up usw. Die Herstellung derartiger Produkte erfolgt auf eine Weise, wie sie dem Fachmann auf diesem Gebiet bekannt ist.

Die neue kosmetische Zusammensetzung mit Bräunungswirkung zeigt einen Effekt, der über das zu erwartende Maß einer länger andauernden Bräunung infolge der Einlagerung in die lamellare Phospholipidstruktur hinausgeht. Die Bräunungswirkung wird gegenüber der DHA-Liposomenstruktur um nahezu das 2-fache verlängert. Darüber hinaus wird eine sehr gleichmäßige Bräunung erzielt, deren Intensität nur langsam nachlässt.

Weiterhin wird der natürliche Farbton der Bräunung besonders verstärkt durch das Vorhandensein von Orangenschalenextrakt. Die Intensität wird durch den Zusatz des Tulpenextraktes verstärkt, wobei Garten- und Wildtulpen der Familie Tulipa bevorzugt sind.

Es wurden Vergleichsversuche durchgeführt, die einen synergistischen Effekt von freiem und lamellar eingeschlossenem DHA sowie solchem mit Orangenschalen extrakt und Tulpenextrakt gegenüber den Einzelkomponenten erkennen lassen.

Die Erfindung soll nachstehend durch Beispiele näher erläutert werden. Alle Angaben erfolgen in Gewichtsprozent, sofern nichts anderes angegeben ist.

### Vergleichsbeispiel 1 Selbstbräunungscreme I

| **Phase A** | |
|---|---|
| Wasser | q.s. ad 100 |
| Glycerin | 2,0 |
| Propylenglycol | 2,0 |

| **Phase B** | |
|---|---|
| Cetearyl Alcohol | 2,6 |
| Steareth-2 | 2,0 |
| Steareth-21 | 1,6 |
| C₁₂₋₁₅ Alkyl Benzoate | 2,1 |
| Dicaprylyl Carbonate | 2,0 |

| **Phase C** | |
|---|---|
| Dihydroxyaceton | 2,0 |
| Dihydroxyaceton lamellar^{*} | 5,0 |
| Tulpenextrakt (sprühgetrocknet) | 1,0 |

| | |
|---|---|
| ^{*} bestehend aus 30% DHA, 6% Phosphatidylcholin, 10% Lipiden, 8% Lösungsmittel und 46% Wasser (Gew-%). | |

Die Phasen A und B wurden nach separater Herstellung bei etwa 65 °C zusammengegeben, etwa 10 Minuten homogenisiert und unter Rühren auf 35 °C abgekühlt. Danach erfolgte die Zugabe der Phase C unter Rühren bei etwa 25 °C. Der pH-Wert wurde auf 4,5 eingestellt.

### Erfindungsgemäße Beispiel Selbstbräunungsgel für Körper und Gesicht

| **Phase A** | |
|---|---|
| Wasser | q.s. ad 100 |
| Glycerin | 3,5 |
| Carbomer | 1,8 |
| Propylenglycol | 3,5 |

| **Phase B** | |
|---|---|
| Dihydroxyaceton | 1,0 |
| Dihydroxyaceton lamellar^{*} | 10,0 |
| Tulpenextrakt (sprühgetrocknet) | 2,0 |
| Orangenextrakt (sprühgetrocknet) | 1,5 |

| **Phase C** | |
|---|---|
| Parfümöl | 0,5 |
| Konservierungsmittel | 0,5 |

| | |
|---|---|
| ^{*} bestehend aus 30% DHA, 6% Phosphatidylcholin, 10% Lipiden, 8% Lösungsmittel und 46% Wasser (Gew-%). | |

Die Phase A wurde unter Rühren auf 40 °C erwärmt. Die separat hergestellte Phase B wurde bei dieser Temperatur unter Rühren zugegeben. Danach wurde Phase C unter Rühren hinzugegeben und der pH-Wert auf pH 4,5 eingestellt.

### Vergleichsbeispiel 2 Selbstbräunungscreme II

| **Phase A** | |
|---|---|
| Wasser | q.s. ad 100 |
| Glycerin | 2,0 |
| Propylenglycol | 2,0 |

| **Phase B** | |
|---|---|
| Cetearyl Alcohol | 2,6 |
| Steareth-2 | 2,0 |
| Steareth-21 | 1,6 |
| C₁₂₋₁₅ Alkyl Benzoate | 2,1 |
| Dicaprylyl Carbonate | 2,0 |

| **Phase C** | |
|---|---|
| Dihydroxyaceton | 1,5 |
| Dihydroxyaceton lamellar^{*} | 2,1 |
| Tulpenextrakt (sprühgetrocknet) | 1,0 |

| | |
|---|---|
| ^{*} bestehend aus 30% DHA, 6% Phosphatidylcholin, 10% Lipiden, 8% Lösungsmittel und 46% Wasser (Gew-%). | |

Es wird wie im Beispiel 1 verfahren.

### Vergleichsbeispiel 3 Selbstbräunungscreme III

| **Phase A** | |
|---|---|
| Wasser | q.s. ad 100 |
| Glycerin - | 2,5 |
| Propylenglycol | 1,9 |

| **Phase B** | |
|---|---|
| Cetearyl Alcohol | 2,6 |
| Steareth-2 | 2,0 |
| Steareth-21 | 1,6 |
| C₁₂₋₁₅ Alkyl Benzoate | 2,1 |
| Dicaprylyl Carbonate | 2,0 |

| **Phase C** | |
|---|---|
| Dihydroxyaceton | 4,2 |
| Dihydroxyaceton lamellar^{*} | 2,8 |
| Tulpenextrakt (sprühgetrocknet) | 0,8 |

| | |
|---|---|
| ^{*} bestehend aus 30% DHA, 6% Phosphatidylcholin, 10% Lipiden, 8% Lösungsmittel und 46% Wasser (Gew-%). | |

Es wird wie im Beispiel 1 verfahren.

### Vergleichsversuche

In einer Probandengruppe von 12 Personen mit Mischhaut wurden auf den Unterarm jedes Probanden zwei Felder von jeweils 2 cm² gekennzeichnet und darauf zwei verschiedene Selbstbräunungscremes aufgetragen. Creme A entsprach der Zusammensetzung von Beispiel 1. Creme B enthielt anstelle von 5% DHA lamellar die gleiche Menge DHA verkapselt in Liposomen.

Unmittelbar nach dem Auftragen wurde der erreichte Farbton mit einer Farbskala vom Tester verglichen. Die Farbskala zeigte Brauntöne von 1 bis 10, wobei 10 der dunkelste Braunton war. Die Messungen sind in Tabelle 1 aufgeführt. Der Unterarm der Testpersonen war im Testzeitraum stets bedeckt und keiner UV-Strahlung ausgesetzt.

Die Ergebnisse in Tabelle 1 zeigen einen Abfall der Brauntönung bei der Creme B vom Mittelwert 8,6 auf 4,4, während bei der Creme A ein Abfall von nur 8,6 auf 7,7 zu erkennen war.

**Tabelle 1**

| **Erreichter Farbton nach Skala 1 - 10** | | | | | | | | |
|---|---|---|---|---|---|---|---|---|
| | **Start** | | **nach 24 h** | | **nach 3 Tagen** | | **nach 7 Tagen** | |
| **Test-personen** | **A** | **B** | **A** | **B** | **A** | **B** | **A** | **B** |
| | | | | | | | | |
| **1** | 9 | 9 | 9 | 8 | 8 | 6 | 8 | 4,5 |
| **2** | 8 | 8 | 8 | 7 | 7 | 5 | 7 | 4 |
| **3** | 9 | 8 | 9 | 8 | 8 | 6 | 7,5 | 5 |
| **4** | 9 | 9 | 9 | 8 | 9 | 7 | 8 | 5 |
| **5** | 9 | 9 | 8 | 8 | 8 | 6 | 8 | 5 |
| **6** | 8 | 9 | 8 | 8 | 8 | 7 | 7 | 4 |
| **7** | 9 | 9 | 9 | 7 | 8 | 5,5 | 8 | 4,5 |
| **8** | 9 | 8 | 8 | 8 | 8 | 6 | 8 | 4 |
| **9** | 9 | 9 | 8 | 7 | 7 | 5 | 7 | 3,5 |
| **10** | 9 | 9 | 9 | 8 | 9 | 7 | 8 | 5 |
| **11** | 9 | 9 | 8 | 7 | 8 | 9 | 8 | 4 |
| **12** | 9 | 8 | 8 | 8 | 8 | 6 | 8 | 4,5 |
| **∅** | **8,8** | **8,6** | **8,4** | **7,7** | **8,0** | **6,0** | **7,7** | **4,4** |

## Patentansprüche

1. Kosmetisches Bräunungsmittel auf Basis von Dihydroxyaceton, **dadurch gekennzeichnet, dass** es enthält
0,1 bis 4,5 Gew-% in eine lamellare Struktur eingelagertes Dihydroxyaceton (DHA),
0,15 bis 3,5 Gew-% freies Dihydroxyaceton,
wobei das Verhältnis zwischen lamellar eingelagertem DHA und freiem DHA im Bereich von 1:1,5 bis 1:0,25 liegt und der Gesamtgehalt an DHA gleich oder kleiner 5 Gew-% ist,
0,01 bis 10 Gew-% Phospholipide,
0,01 bis 5 Gew-% eines alkoholischen Extraktes von Orangenschalen im Gemisch mit 0,5 bis 8 Gew-% eines alkoholischen Extraktes von Tulpen und
bis 100 Gew-% kosmetische Hilfsstoffe, Trägerstoffe, weitere Wirkstoffe und Gemische davon,
wobei alle Anteile auf das Gesamtgewicht des Bräunungsmittels bezogen sind.

2. Bräunungsmittel nach Anspruch 1, **dadurch gekennzeichnet, dass** es das in eine lamellare Struktur eingelagerte DHA als ein Gemisch von Phospholipiden, Lipiden, organischen Lösungsmitteln, Wasser und DHA in einem Anteil von 4-12 Gew-% enthält.

3. Bräunungsmittel nach Anspruch 1, **dadurch gekennzeichnet, dass** es 1 bis 4,0 Gew-% in eine lamellare Struktur eingelagertes Dihydroxyaceton (DHA) enthält.

4. Bräunungsmittel nach Anspruch 1, **dadurch gekennzeichnet, dass** es 0,5 bis 2,0 Gew-% freies Dihydroxyaceton enthält.

5. Bräunungsmittel nach Anspruch 1, **dadurch gekennzeichnet, dass** es 0,1 bis 1,5 Gew-% in eine lamellare Struktur eingelagertes DHA und 0,15 bis 2,5 Gew-% freies DHA enthält.

6. Bräunungsmittel nach Anspruch 1, **dadurch gekennzeichnet, dass** es zusätzlich einen Süßholzextrakt (Liquorice) mit einem Anteil von 0,001 bis 1 Gew-% enthält.

## Claims

1. A cosmetic bronzing agent based on dihydroxyacetone comprising 0.1 to 4.5 wt. % dihydroxyacetone (DHA) incorporated in a lamellar structure,
0.15 to 3.5 wt. % free dihydroxyacetone,
the ratio of DHA incorporated in lamellae and free DHA ranging between 1:1.5 and 1:0.25 and the total amount of DHA contained being equal to or less than 5 wt. %,
0.01 to 10 wt. % phospholipids,
0.01 to 5 wt. % of an alcoholic extract from orange peels mixed with 0.5 to 8 wt. % of an alcoholic extract from tulips,
and
cosmetic auxiliary agents, carrier substances, additional active ingredients and mixtures thereof up to 100 wt. %,
all percentages being relative to the bronzing agent's total weight.

2. A bronzing agent according to claim 1, wherein said agent contains DHA incorporated in a lamellar structure as a mixture of phospholipids, lipids, organic solvents, water and DHA in an amount of 4-12 wt. %.

3. A bronzing agent according to claim 1, wherein said agent contains 1 to 4.0 wt. % dihydroxyacetone (DHA) incorporated in a lamellar structure.

4. A bronzing agent according to claim 1, wherein said agent contains 0.5 to 2.0 wt. % free dihydroxyacetone.

5. A bronzing agent according to claim 1, wherein said agent contains 0.1 to 1.5 wt. % DHA incorporated in a lamellar structure and 0.15 to 2.5 wt. % free DHA.

6. A bronzing agent according to claim 1, wherein said agent additionally contains a liquorice extract in an amount ranging between 0.001 and 1 wt. %.

## Revendications

1. Produit bronzant à base de dihydroxyacétone, **caractérisé en ce qu'**il contient
0,1 à 4,5 % en poids d'une dihydroxyacétone (DHA) stockée dans une structure lamellaire,
0,15 à 3,5 % en poids de dihydroxyacétone libre,
le rapport entre la DHA stockée au niveau lamellaire et la DHA libre étant de l'ordre de 1:1,5 à 1:0,25 et la teneur totale en DHA étant inférieure ou égale à 5 % en poids,
0,01 à 10 % en poids de phospholipides,
0,01 à 5 % en poids d'un extrait alcoolique d'écorce d'orange dans le mélange avec 0,5 à 8 % en poids d'un extrait alcoolique de tulipe, et
jusqu'à 100 % en poids d'adjuvants cosmétiques, de véhicules, d'autres principes actifs et leurs mélanges,
toutes les parties se rapportant au poids total du produit bronzant.

2. Produit bronzant selon la revendication 1, **caractérisé en ce qu'**il contient la DHA stockée dans une structure lamellaire en tant que mélange de phospholipides, lipides, solvants organiques, eau et DHA en une proportion de 4 à 12 % en poids.

3. Produit bronzant selon la revendication 1, **caractérisé en ce qu'**il contient 1 à 4,0 % en poids de dihydroxyacétone (DHA) stockée dans une structure lamellaire.

4. Produit bronzant selon la revendication 1, **caractérisé en ce qu'**il contient 0,5 à 2,0 % en poids de dihydroxyacétone libre.

5. Produit bronzant selon la revendication 1, **caractérisé en ce qu'**il contient 0,1 à 1,5 % en poids de DHA stockée dans une structure lamellaire et 0,15 à 2,5 % de DHA libre.

6. Produit bronzant selon la revendication 1, **caractérisé en ce qu'**il contient en outre un extrait de réglisse (liquorice) en une proportion de 0,001 à 1 % en poids.
